# EUROPEAN PATENT SPECIFICATION

(11) **EP 2 606 854 B1**
(45) Date of publication and mention of the grant of the patent: **19.10.2016**
(21) Application number: 12275202.5
(22) Date of filing: 13.12.2012
(51) Int. Cl.: A61F 2/07, A61F 2/852

(54) **Low profile non-symmetrical stents and stent grafts**
Asymmetrische Niederprofil-Stents und Stentimplantate
Stents asymétriques à profil bas et greffons de stents

(30) Priority: 22.12.2011 US 201113335142
(43) Date of publication of application: 26.06.2013
(73) Proprietor: Cook Medical Technologies LLC, Bloomington, IN 47404 (US)
(72) Inventor: Chuter, Timothy, San Francisco, CA 94114 (US); Roeder, Blayne, Lafayette, IN 47909 (US)
(74) Representative: Georgiou, Sarah Caroline

(56) References cited:
- EP-A1- 0 960 607
- WO-A1-2012/176187
- US-A1- 2003 120 331
- US-A1- 2004 111 146
- US-A1- 2004 176 833
- US-A1- 2007 150 051

## Description

### BACKGROUND

The present embodiments relate generally to stents and stent-grafts for use in body vessels to treat medical conditions.

Stents may be inserted into an anatomical vessel or duct for various purposes. Stents may maintain or restore patency in a formerly blocked or constricted, passageway, for example, following a balloon angioplasty procedure. Other stents may be used for different procedures, for example, stents placed in or about a graft have been used to hold the graft in an open configuration to treat an aneurism. Additionally, stents coupled to one or both ends of a graft may extend proximally or distally away from the graft to engage a healthy portion of a vessel wall away from a diseased portion of an aneurysm to provide endovascular graft fixation.

Stents may be either self-expanding or balloon-expandable, or they can have characteristics of both types of stents. Various existing self-expanding and balloon-expandable stent designs and configurations comprise generally symmetrical end regions including one or more apices formed of nitinol or another alloy wire formed into a ring. The apices commonly comprise relatively acute bends or present somewhat pointed surfaces, which may facilitate compression of the stent to a relatively small delivery profile due to the tight bend of the apices. Although having this advantage, in some situations, such relatively acute or pointed apices may be undesirable, in particular in vessel anatomies that are curved or tortuous such as, for example, the thoracic aorta.

The thoracic aorta presents a challenging anatomy for stent grafts used to treat. thoracic aneurysms or dissections. The thoracic aorta comprises a curve known as the aortic arch, which extends between the ascending thoracic aorta (closet to the heart) and the descending thoracic aorta (which extends toward the abdominal aorta). Thoracic stent grafts are used to exclude thoracic aortic aneurysms. A stent graft's ability to conform to the tortuous anatomy of the aortic arch is a major concern. Current designs sometimes lack the desired sealing ability at the proximal end of the stent graft (closest to the heart). Also, current thoracic devices present a relatively large profile which, with some patients' anatomies may be problematic. Finally, many current stents have relatively acute points that may prevent them from being used in the aortic arch for fear of undesirable interaction with the artery wall after an extended amount of time in the patient.

Therefore, a generally nonsymmetrical stent having at least one relatively rounded apex that is less invasive in an expanded state than stents with more acute apices may alleviate the above problems, while providing an improved compliance to the aortic arch and increased radial force if used as a sealing and/or alignment stent, as well as a desirable ability to be crimped to a readily introducible diameter.

As one particular example, type-A thoracic aortic dissection (TAD-A) is a condition in which the intimal layer of the ascending thoracic aorta develops a tear, allowing blood to flow into the layers of the aortic wall, causing the development of a medial or subintimal hematoma. TAD-A is associated with a strikingly high mortality rate (about one-fourth to one-half of victims die within the first 24-48 hours). The only current treatment for TAD-A is open surgery, where the chest is opened, the aorta is clamped, and a vascular prosthesis is sewn in place. Operative mortality rate for this procedure may be around 10%. Endovascular treatment of TAD-B (which affects the descending thoracic aorta) has been effective in reducing short-term and longer term mortality. Therefore, it is desirable to provide an endovascular device configured to address the anatomic challenges of the thoracic aorta.

EP 0960607 describes a stent graft assembly including caps on either or both ends for improved and uniform deployment of the assembly.

US 2007/150051 describes a vascular implant constructed of a single woven wire to form at least a main lumen having proximal and distal ends.

US 2004/111146 describes an apparatus and method for attachment between intersecting stent members and a covering or graft. The attachment fixes the covering relative to selected intersections of the stent members, preventing the covering from wrinkling etc. during expansion of the stent.

### SUMMARY

Various stents and stent-grafts for treatment of medical conditions are disclosed. Aspects of the present invention are described in the appended claims. In one example, a stent-graft comprises a graft and first and second stents rings. The first stent ring comprises a plurality of proximal apices, a plurality of distal apices, and a plurality of generally straight portions disposed between the plurality of proximal and distal apices. The second stent ring comprises a plurality of proximal apices, a plurality of distal apices, and a plurality of generally straight portions disposed between the plurality of proximal and distal apices.

The series of distal apices of the first stent ring are each disposed distal to the proximal end of the graft, and the series of proximal apices of the first stent ring are each disposed proximally beyond the proximal end of the graft. Additionally, the series of distal apices of the second stent ring are each disposed distal to the proximal end of the graft, and the series of proximal apices of the second stent ring are each disposed proximally beyond the proximal end of the graft.

The first stent ring comprises a first uniform segment, and the second stent ring comprises a second uniform wire segment. The first uniform segment comprises portions disposed both internal and external to the second uniform wire segment.

In one example, each of the proximal apices of the first stent ring may be circumferentially offset from each of the proximal apices of the second stent. Further, each of the distal apices of the first stent ring may be circumferentially offset from each of the distal apices of the second stent.

In one example, the first stent ring and the second stent ring comprise identical geometries. At least one distal apex of the first stent ring may comprise a first curved portion having a first radius of curvature, and at least one proximal apex of the first stent ring may comprise a second curved portion having a second radius of curvature, where the second radius of curvature is greater than the first radius of curvature. Similarly, at least one distal apex of the second stent ring comprises a third curved portion having a third radius of curvature, and at least one proximal apex of the second stent ring comprises a fourth curved portion having a fourth radius of curvature, where the fourth radius of curvature is greater than the third radius of curvature.

Other systems, methods, features and advantages of the invention will be, or will become, apparent to one with skill in the art upon examination of the following figures and detailed description. It is intended that all such additional systems, methods, features and advantages be within the scope of the invention, and be encompassed by the following claims.

### Brief Description Of The Drawings

Embodiments of the present invention are described below, by way of example only, with reference to the accompanying drawings. The components in the figures are not necessarily to scale, emphasis instead being placed upon illustrating the principles of the invention. Moreover, in the figures, like referenced numerals designate corresponding parts throughout the different views.
FIGS. 1-3 show different views of a symmetrical stent;
FIG. 4 depicts an example of an asymmetric stent;
FIG. 5 diagrammatically illustrates the asymmetrical radii of curvature of the stent of FIG. 4;
FIG. 6 shows the stent of FIG. 4 in a simulated artery;
FIG. 7 depicts another example of an asymmetric stent;
FIG. 8 diagrammatically illustrates the asymmetrical radii of curvature of yet another example of a stent;
FIG. 9 shows the stent of FIG. 8 in a simulated artery;
FIG. 10 shows an end view of still another example of an asymmetric stent;
FIG. 11 shows a side view of the stent of FIG. 10;
FIG. 12 is a top perspective view of the stent of FIG. 10; FIG. 13 shows the stent of FIG. 10 in a simulated artery;
FIG. 14 is a partial perspective of a stent-graft incorporating the stent of FIG. 10;
FIG. 15 illustrates a side view of the stent-graft of FIG. 14;
FIGS. 16-18 show a stent-graft with side branches;
FIG. 19 is a side view of a stent-graft device configured for endovascular treatment of a thoracic aorta dissection; and
FIGS. 20-21 are, respectively, side and perspective views of a proximal region of an alternative stent-graft.

### DETAILED DESCRIPTION OF THE PREFERRED EMBODIMLNTS

The present embodiments relate generally to stents and stent-grafts for use in body vessels to treat medical conditions. In particular, the embodiments relate to a novel asymmetric stent having opposing sets of curved apices, where the curved section of one set of apices has a radius of curvature that is greater than the curved section of the other set of apices, and may present a lower profile than conventional stents. The lower profile may present advantages for use in patients with particularly tortuous or small-diameter vessels.

In the present application, the term "proximal" refers to a direction that is generally closest to the heart during a medical procedure, while the term "distal" refers to a direction that is furthest from the heart during a medical procedure. Reference throughout is made to proximal and distal apices, but those of skill in the art will appreciate that the proximal-distal orientation of stents of the present invention may be reversed without exceeding the scope of the present invention.

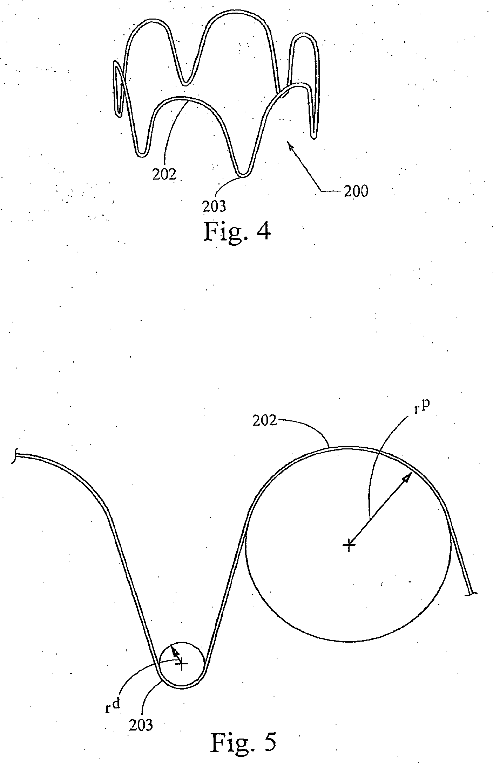

As shown in Figures 4-15, this novel stent is not symmetrical like many commercially available stents, in that the radius of curvature of the opposing proximal and distal apices is different between the top and bottom of the stent. The stents may be attached to either end of a stent graft to provide sealing and may be used internally or externally to the graft material to provide support to the graft.

The asymmetric stent may be configured such that, when used with a graft, it will provide a sufficiently strong radial force at the graft's end openings to hold the graft material open against the artery wall. Also, the stent is intended to be short in length so that the graft will include flexibility sufficient to accommodate a patient's anatomy. This combination of flexibility and strong radial force provides an improved seal between the graft and artery wall. In addition, enhanced flexibility is provided as well, particularly when one or more stents are used to provide short segments and better accommodate curves.

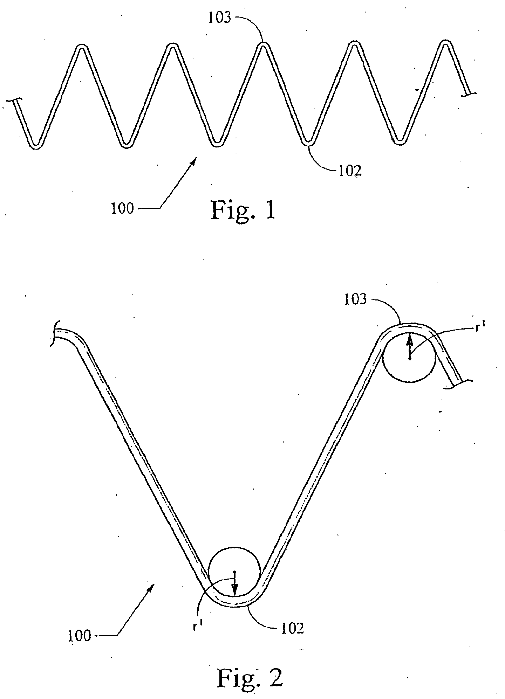

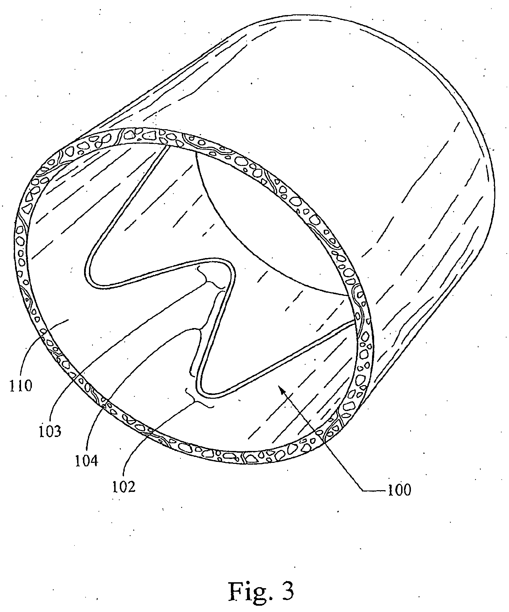

FIG. 1 shows a conventional stent 100, which has symmetrical apices 102, 103. Specifically, the proximal apices 102 and the distal apices 103 all have generally the same radii of curvature (r¹), which is illustrated in graphic form in FIG. 2. FIG. 3 is adapted from an FEA contour simulation and shows the stent 100 in a simulated artery 110, where the stent 100 is 20% oversized. The proximal and distal apices 102, 103 (circled) exert little or no pressure against the artery wall 110, while an intermediate region 104 exerts a higher pressure to provide - in one example - a total radial sealing force of 0.178 lbf (equivalent to approximately 0.79N). This configuration may be crimped to 18 Fr (*e.g.,* for introduction via a catheter), with a maximum bend strain in the apices 102, 103 of about 5.8%. When using, for example, a typical NiTi wire for the stent, it is desirable not to exceed 10-12% strain to avoid increased risk of deforming the wire or adversely affecting its durability.

FIGS. 4-7 show a first example of a non-symmetrical stent 200, which is formed as a wire ring that has non-symmetrical proximal and distal generally curved apex portions (apices) 202, 203 separated from each other by intermediate generally straight portions. Specifically, the distal apices 203 all have generally the same radii of curvature (r^{d}) as each other, but the distal apices' radii of curvature are different from those of the proximal apices 202 (r^{p}). The distal apices 203 (which may be attached to stent-grafts as described below) are generally narrowly rounded in a manner not dissimilar from a traditional z-stent, but the proximal apices 202 are more broadly rounded. The difference in the proximal and distal apices 202, 203 is illustrated in graphic form in FIG. 5. In the illustrated example, the rounded proximal apices 202 have a radius of curvature of 6.0 mm, while the narrower distal apices 202 have a radius of curvature of 1.0 mm. In certain examples of non-symmetrical stents, the radius of curvature of the rounded proximal apices (measured in the manner shown in FIG. 5) may be from about 4 mm to about 9 mm, and the radius of curvature of the narrower distal apices may be from about 0.5 mm to about 1.5 mm.

In these and other examples, the ratio of the proximal apices' radius of curvature to the distal apices' radius of curvature may be about 2.6:1 to about 18:1, and desirably may be about 6:1. The outer circumference of the stent 200 preferably is generally consistent such that, in this configuration, a solid outer face around the stent 200 would form a cylinder, although the stent will most preferably provide compliance with a surface less smooth than a cylinder.

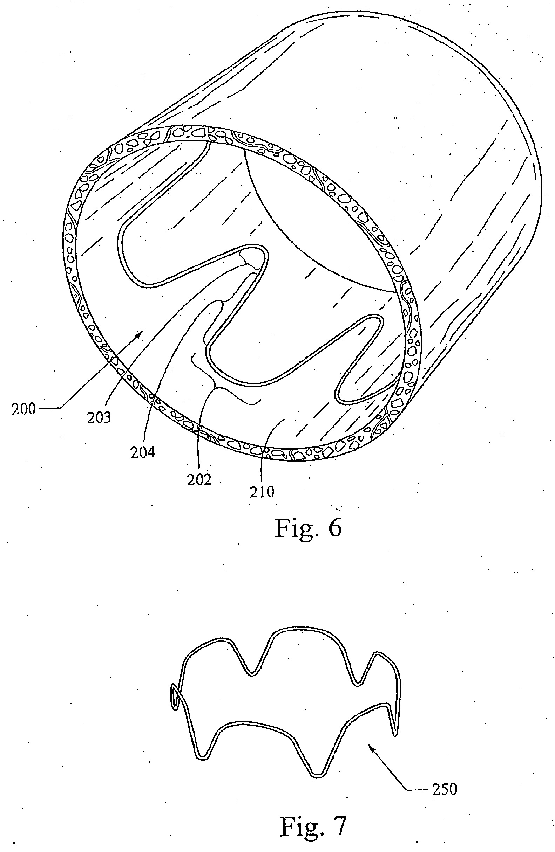

FIG. 6 is adapted from an FEA contour simulation and shows the stent 200 in a simulated artery 210, where the stent 200 is 20% oversized. The proximal and distal apices 202, 203 (circled) exert little or no pressure against the artery wall 210, while an intermediate region 204 (boxed) exerts a greater pressure to provide - in the illustrated example - a total radial sealing force of about 0.160 lbf (equivalent to approximately 0.47N). This configuration may be crimped to 18 Fr, with a maximum bend strain in the apices 202, 203 of about 6.5%.

FIG. 7 shows another non-symmetrical stent embodiment 250 that is very similar to the embodiment of FIGS. 4-6, but which has a shorter proximal-distal length. Each of the examples shown in FIGS. 4-7 may be manufactured in substantially the same manner as current z-stents, with a modification only of forming the proximal apices to include a greater radius of curvature than the distal apices.

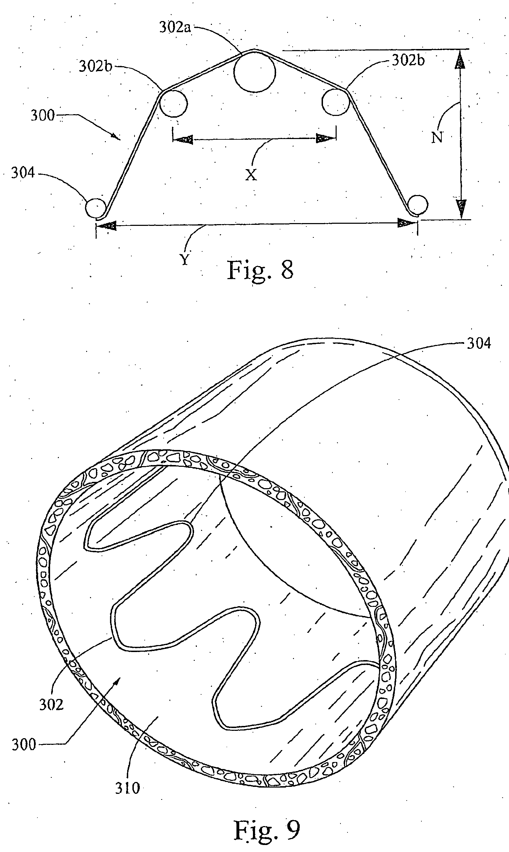

FIGS. 8-9 illustrate another example of a non-symmetrical stent 300, which has a proximal "rounded roof shape" profile rather than the generally semicircular profile of the examples described above with reference to FIGS. 4-7. The profile of each proximal apex 302 includes a central fillet 302a and a pair of symmetrically opposed shoulder fillets 302b that may be generally equidistant from the central fillet 302a, or that may be disposed at varied distances therefrom. For the proximal apices of the stent 300, the central fillets 302a each have a radius of curvature of 1.0 mm, and the shoulder fillets 302b each have a fillet radius of curvature of 0.5 mm. The distal apices 304 have a radius of curvature of 1.0 mm. In another example having the rounded roof shape configuration (not shown), the central and shoulder fillets of proximal apices may each have the same radius of curvature such as, for example, 0.5 mm each, with distal apices also having a 0.5 mm radius of curvature. In other examples, the central and shoulder fillets 302a, 302b may each have a radius of curvature from about 0.5 mm to about 5 mm, and the distal apices may each have a radius of curvature of about 0.5 mm to about 1.5 mm. In another example having the rounded roof shape configuration (not shown), the ratio between the radii of curvature of the central and each shoulder fillet of the proximal apices may be about 3:1. FIG. 8 also shows three spans useful for describing desirable proportions in stent embodiments: "x" indicates the distance between the apical extremities of the shoulder fillets 302b, "y" indicates the distance between the tips of the distal apices 304, and "z" indicates the distance along a longitudinal axis between the tip of the distal apices 304 and the apical extremity of the proximal fillet 302a. Desirable embodiments may include an x:y ratio of about 1:3 to about 7:8 and a y:z ratio of about 1:1 to about 3: 1. In yet another example (not shown), the filleted apices of this example may be combined with the generally semicircular apices of the example described with reference to FIGS. 4-7.

FIG. 9 is adapted from an FEA contour simulation and shows the stent 300 in a simulated artery 310, where the stent 300 is 20% oversized. The proximal and distal apices 302, 304 exert little or no pressure against the artery wall 310, while an intermediate region exerts a greater pressure to provide - in the illustrated example - a total radial sealing force of about 0.420 lbf (equivalent to approximately 1.87N). This configuration may be crimped to 18 Fr, with maximum bend strains in the apices that may be less than about 9% and preferably are less than about 10-12%. The greater radial sealing force of this example may provide advantages for stent placement and retention in certain circumstances as compared to existing z-stents.

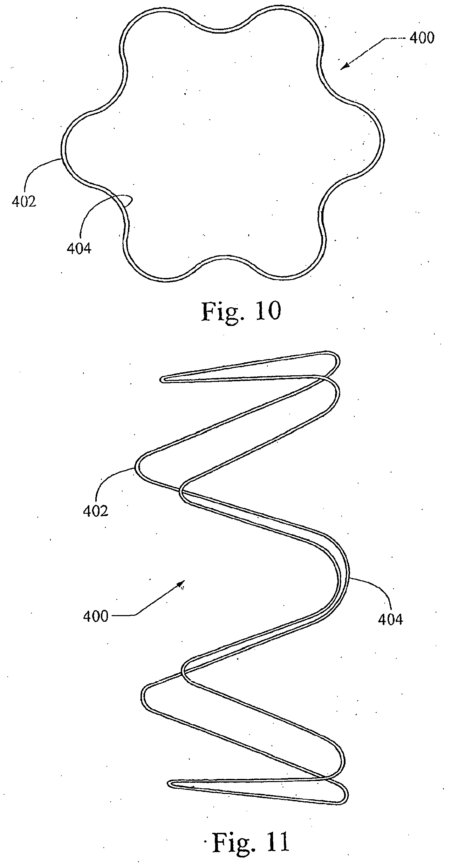

FIGS. 10-13 illustrate another example of a non-symmetrical stent 400, which has an expanded "flower configuration" as shown in FIG 10. Specifically, when the stent 400 is in an expanded configuration, the circumference around the proximal more-rounded apices 402 is greater than the circumference around the distal less-rounded apices 404, which is shown most clearly in FIGS. 11-14. In this configuration a solid outer face around an expanded stent 400 would form a frustum of a cone. This configuration may be manufactured in the same manner as the examples described above with reference to FIGS. 4-7 (*i.e.,* producing a stent with a generally uniform outer circumference), with an added step that may include drawing the distal apices 404 into a smaller circumference upon suturing them to a smaller diameter graft material. Alternatively, or in addition, the stent 400 may be heat-set to impose the desired shape.

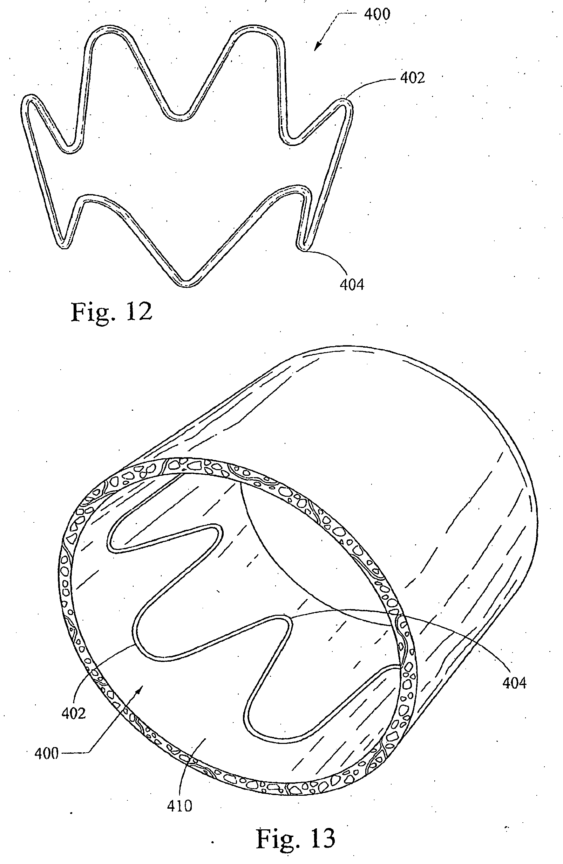

FIG. 13 is adapted from an FEA contour simulation and shows the stent 400 in a simulated artery 410, where the stent 400 is 20% oversized. Surprisingly, the contour of pressure distribution along proximal and distal apices 402, 404 as well as an intermediate region is generally uniform throughout the stent circumference. The illustrated configuration provides a total radial sealing force of about 0.187 lbf. This property of generally uniform pressure distribution may provide advantages in certain applications of providing a seal and/or presenting less abrasion of a vessel wall through graft material as compared to stents, with less uniform pressure distribution.

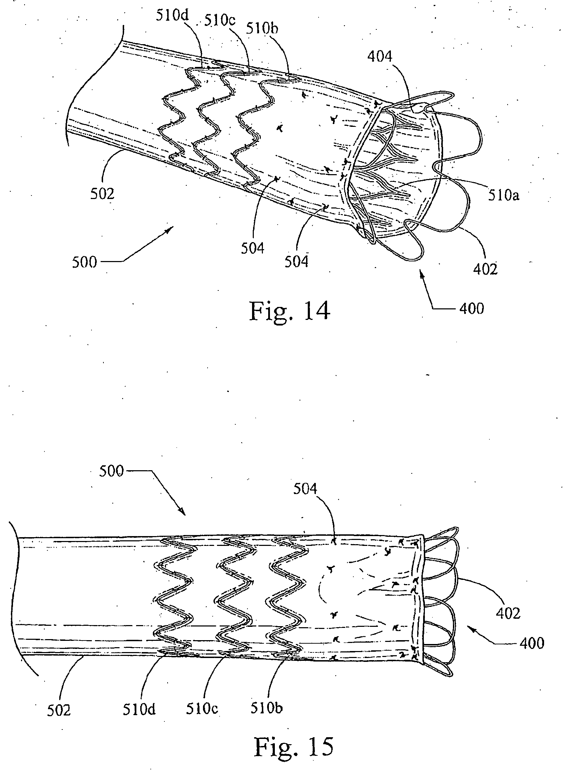

FIGS. 14-15 show two different views of a stent graft 500 using a stent example 400 of the present invention described above with reference to FIGS. 10-13. The stent graft 500 is shown in an expanded state and may be configured for use in treating a thoracic aortic aneurysm. The stent 400 is disposed at the proximal end of a generally cylindrical graft sleeve 502, to which its distal apices 404 are secured by sutures 504. The stent-graft'500 also includes a series of z-stents 510a-d disposed distally from the stent 400. The first z-stent 510a is attached to the inner circumference of the graft 502, and the other z-stents 510b-510d are attached to the outer diameter of the graft 502. The proximal end of the stent 400 extends beyond the proximal end of the graft in a manner that may facilitate anchoring the graft in a vessel of a patient (e.g., a blood vessel). - ,

The rounded points on the stent may protrude from the graft material only a small amount as is shown in FIGS. 14-15. In this example, only a small portion of the bare wire will be exposed to the artery wall. These unique (larger radii) rounded points are far less likely to perforate the artery wall than sharper points of a different stent configuration. Advantageously, this asymmetric stent design will maximize the efficacy of the seal while preserving the condition of the artery wall. Specifically, the narrower stent apices will provide for desirable radial expansion/ sealing force, and the broader rounded apices will provide for a desirably atraumatic contact with an artery wall.

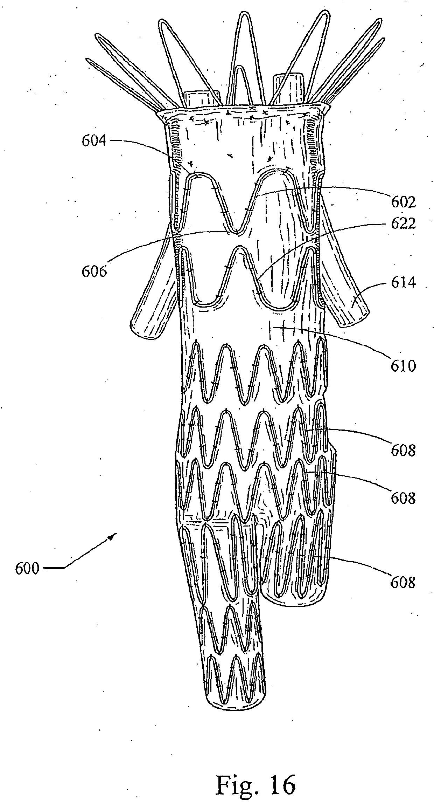

FIGS. 16-18 show a stent-graft embodiment 600 that includes a non-symmetrical stent 602 having more broadly rounded proximal apices 604 and more narrowly rounded distal apices 606. The stent 602 is attached by sutures to the inner surface (not shown) or outer surface of a generally columnar graft 610, which includes other stents 608. A second layer of graft material 612 is also attached to the inner circumference of the graft 610 midway down its length and extends proximally through the inner circumference of the stent 602.

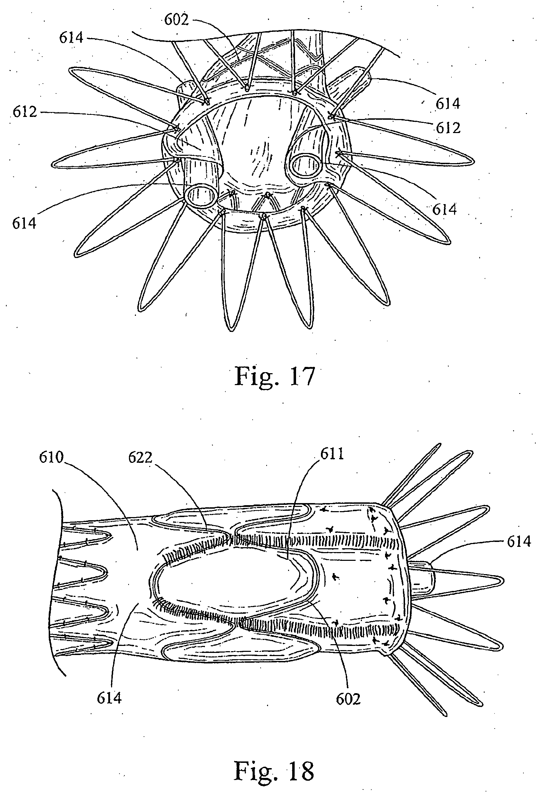

As shown in the end view of FIG. 17, this construction provides a passage for branch structures 614 (that may be embodied, for example, as tubular or non-tubular stents, stent-grafts, shown here for the sake of illustration as generic tubular structures), which pass through the passage formed between the two layers 610, 612 and through an aperture 611 in the graft 610. The tubular structures 614 will advantageously be disposed generally transversely through the inner radius of the more broadly rounded proximal apices 604 of the stent 602, which provides atraumatic columnar support for the graft 610 as well as an anchor for the tubular structures 614. The stent-graft 600 may be particularly useful for treatment of an abdominal aortic aneurysm (AAA) that is immediately adjacent to, or that goes across, the renal arteries such that it has a short neck and lacks a contact area that is sufficient to create an effective proximal seal and avoid the proximal Type I endo leaks that may occur with some currently-available AAA stent-grafts. Those of skill in the art will appreciate that the stent-graft 600 will allow general occlusion of the AAA, while providing patent passage through the descending aorta and from the aorta to the renal arteries. Specifically, a stent-graft configured in the manner of the stent-graft embodiment 600, which includes a modular design that may include branch stents and/or stent-grafts, will allow a seal to be formed above the renal arteries and below the celiac and superior mesenteric arteries. Also, as shown in FIG. 16, a second non-symmetrical stent 622 may be placed adjacent the first non-symmetrical stent 602 in an opposite orientation that will provide additional atraumatic support for the branching tubular structures 614.

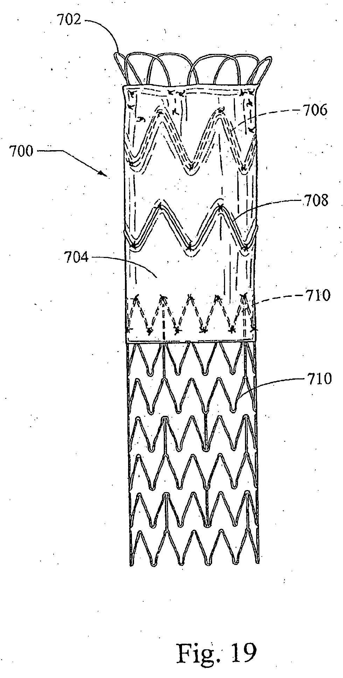

FIG. 19 shows a stent-graft device 700 .configured for endovascular treatment of a thoracic aorta dissection. The device 700 includes a non-symmetrical alignment stent 702 attached to a first end of a tubular graft material 704. A sealing stent 706 is attached in the central lumenal graft space proximate the alignment stent 702. The sealing sterit 706 preferably is configured with a high radial force to promote efficacious sealing of the graft material 704 against a vessel wall. A body stent 708 configured here as a z-stent is disposed on the exterior of the graft material 704 and preferably is configured to provide longitudinal and circumferential stability/ columnar support for the graft material of the device 700, such that it will conform to the vasculature and resist buckling when deployed in torturous anatomy such as the ascending thoracic aorta. A bare cannula stent 710 (such as, for example, a cut nitinol stent) is attached in the tubular graft material 704 at the opposite end from the alignment stent 702. This cannula stent 710 preferably is a conformable kink-resistant stent that provides distal sealing and migration-resistance. In a deployment of the device 700 to treat an aortic dissection, the alignment stent 702 preferably will be disposed proximal (nearer the heart) relative to the vessel tear, with the graft material traversing the tear in a manner generally sealing it from blood flow. And, the distal cannula stent 710 will help conform to the vasculature and retain a seal for treatment of the dissection. One or.more of the sealing stent 706, body stent 708, and bare stent 710 may include one or more barbed projections configured to help anchor the device 700.

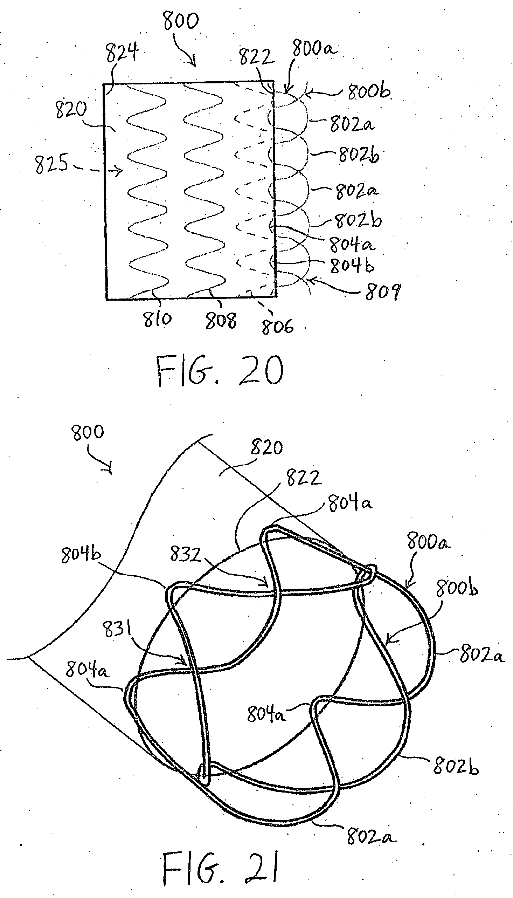

Referring now to FIGS. 20-21, an alternative stent-graft 800 comprises a graft 820 having proximal and distal ends 822 and 824 and a lumen 825 extending therebetween, and also comprises a first stent 800a and a second stent 800b. In this non-limiting embodiment, the first stent 800a comprises a plurality of proximal apices 802a, a plurality of distal apices 804a, and a plurality of generally straight portions disposed between the plurality of proximal and distal apices 802a and 804a. Similarly, the second stent 800b comprises a plurality of proximal apices 802b, a plurality of distal apices 804b, and a plurality of generally straight portions disposed between the plurality of proximal and distal apices 802b and 804b, as shown in FIGS. 20-21.

In one embodiment, the series of distal apices 804a of the first stent 800a are each disposed distal to the proximal end 822 of the graft 820, and the series of proximal apices 802a of the first stent 800a are each disposed proximally beyond the proximal end 822 of the graft 820. Additionally, the series of distal apices 804b of the second stent 800b are each disposed distal to the proximal end 822 of the graft 820, and the series of proximal apices 802b of the second stent 800b are each disposed proximally beyond the proximal end 822 of the graft 820, as shown in the illustrative embodiment of FIGS. 20-21. It should be noted that the series of distal apices 804a and 804b of the first and seconds stents 800a and 800b, respectively, may be coupled to either the inside or outside of the graft 820.

The first stent 800a may comprise a first uniform segment, and the second stent 800b may comprise a second uniform segment, as depicted in FIGS. 20-21. In one example, the first uniform segment of the first stent 800a comprises portions disposed both internal and external to the second uniform segment of the second stent 800b, as noted by overlapping regions 831 and 832 in FIG. 21. The first stent 800a and the second stent 800b may comprise a single filament of wire that is wound into the desired configuration, or may originate from a cannula that is formed, e.g., by laser cutting, into the configuration shown.

In one embodiment, each of the proximal apices 802a of the first stent 800a may be circumferentially offset from each of the proximal apices 802b of the second stent 800b. Further, each of the distal apices 804a of the first stent 800a may be circumferentially offset from each of the distal apices 804b of the second stent 800b.

In one exemplary embodiment, the first stent 800a and the second stent 800b comprise identical geometries. By way of example, and without limitation, the first stent 800a and the second stent 800b may comprise similar features to the stents 200 and 702, described in detail above. In this embodiment, at least one distal apex 804a of the first stents 800a may comprise a first curved portion having a first radius of curvature, e.g., (r^{d}) in the embodiment of FIG. 5 above, and at least one proximal apex 802a of the first stent 800a may comprise a second curved portion having a second radius of curvature, e.g., (r^{p}) in the embodiment of FIG. 5 above, where the second radius of curvature is greater than the first radius of curvature. Similarly, at least one distal apex 804b of the second stent 800b comprises a third curved portion having a third radius of curvature, and at least one proximal apex 802b of the second stent 800b comprises a fourth curved portion having a fourth radius of curvature, where the fourth radius of curvature is greater than the third radius of curvature.

In one embodiment, the dimensions of the third radius of curvature of the second stent 800b may correspond to the dimensions of the of the first radius of curvature of the first stent 800a, while the dimensions of the fourth radius of curvature of the second stent 800b may correspond to the dimensions of the of the second radius of curvature of the first stent 800a. In alternative embodiments, it is contemplated that the first and third radii of curvature may differ relative to each other, and the second and fourth radii of curvature may differ relative to each other.

In the instance in which the first stent 800a and the second stent 800b comprise similar features to the stents 200 and 702 described in detail above, the second radius of curvature of at least one of the proximal apices 802a of the first stent 800a is at least two times greater than the first radius of curvature of at least one of the distal apices 802a. In one example, the first radius of curvature is from about 0.5 mm to about 1.5 mm, while the second radius of curvature is from about 4 mm to about 9 mm. A ratio of the first radius of curvature to the second radius of curvature may be about 1:2.6 to about 1:l 8.

Advantageously, in the embodiment of FIGS. 20-21, the provision of the first and second stents 800a and 800b in the manner depicted may provide an increased rounded profile at the proximal end that contacts a vessel wall, thereby potentially reducing trauma to a patient's vessel. More specifically, by providing two stents 800a and 800b, each having relatively rounded proximal apices, and arranging them in an out-of-phase alignment, the number of non-contact spaces 809 may be reduced, while rounded regions for atraumatic contact are enhanced.

Notably, if the first and second stents 800a and 800b overlap with one another as shown in FIGS. 20-21, the number of trigger wires does not need to be increased, and in fact may be reduced, since radially restraining one portion of one of the stents 800a or 800b will also radially restrain portions of the other stent. Further, it should be noted that if first and second stents 800a and 800b are used in an overlapping manner, the wires forming the first and second stents 800a and 800b may comprise smaller cross-sectional areas, and therefore the overall radial profile of the device may not be increased. In certain embodiments, the radial force provided by the first stent 800a may be greater or less than the radial force provided by the second stent 800b.

In the embodiment of FIGS. 20-21, any number of additional stents, beyond the first and second stents 800a and 800b, may be used depending on the nature of the condition and bodily passage. In this non-limiting example, the stent-graft 800 comprises a sealing stent 806 attached to the graft 820 within the lumen 825 distal to the proximal end 822 of the graft 820, as shown in FIG. 20 (the sealing stent 806 is omitted for illustrative clarity in FIG. 21). Additional, exemplary stents 808 and 810 are disposed distal to the sealing stent 806, though it will be appreciated that any number of stents may be coupled to the graft 820.

While various embodiments of the invention have been described, the invention is not to be restricted except in light of the attached claims and their equivalents. Moreover, the advantages described herein are not necessarily the only advantages of the invention and it is not necessarily expected that every embodiment of the invention will achieve all of the advantages described.

This application claims the benefit of priority of U.S. Utility Publication No. 2012-0130479, entitled "Low Profile Non-Symmetrical Stents and Stent-Grafts," filed December 22, 2011, which is a continuation-in-part of U.S. Utility Patent Publication Numbed 2010-0161026, filed November 19, 2009 and entitled "Low Profile Non-Symmetrical Stent," which claims priority to U.S. Provisional Application Ser. No. 61/016,753, filed December 26, 2007, co-pending U.S. Patent Publication Nos. 2009-0171437, filed December 11, 2008 and 2009-036763, filed May 26, 2009, Great Britain Patent No. GB2475494, filed November 18, 2009, and co-pending Great Britain Patent Publication No. GB2476451, filed November 19, 2009.

## Claims

1. A stent-graft (800) for use in a medical procedure, the stent-graft comprising:
a graft having proximal and distal ends (822, 824) and a lumen (825) extending therebetween;
a first stent ring (800a) comprising a plurality of proximal apices (802a), a plurality of distal apices (804a), and a plurality of generally straight portions disposed between the plurality of proximal and distal apices, where the series of distal apices (804a) of the first stent ring are each disposed distal to the proximal end (822) of the graft, and where the series of proximal apices (802a) of the first stent ring are each disposed proximally beyond the proximal end (822) of the graft; and
a second stent ring (800b) comprising a plurality of proximal apices (802b), a plurality of distal apices (804b), and a plurality of generally straight portions disposed between the plurality of proximal and distal apices, where the series of distal apices (804b) of the second stent ring are each disposed distal to the proximal end (822) of the graft, and where the series of proximal apices (802b) of the second stent ring are each disposed proximally beyond the proximal end (822) of the graft;
wherein the first (800a) and second (800b) stent rings are out of phase;
where the first stent ring (800a) comprises a first uniform segment, and the second stent ring (800b) comprises a second uniform segment, where the first uniform segment comprises portions disposed both internal and external to the second uniform segment and where the first and second stent rings overlap relative to one another; and
wherein the overlapping first (800a) and second (800b) stent rings are located only at the proximal end of the graft.

2. The stent-graft of claim 1 where the first stent ring (800a) and the second stent ring (800b) comprise identical geometries.

3. The stent-graft of any preceding claim where at least one distal apex (804a) of the first stent ring (800a) comprises a first curved portion having a first radius of curvature, and at least one proximal apex (802a) of the first stent ring (800a) comprises a second curved portion having a second radius of curvature, where the second radius of curvature is greater than the first radius of curvature.

4. The stent-graft of claim 3 where the second radius of curvature is at least two times greater than the first radius of curvature.

5. The stent-graft of any of claims 3 and 4 where the first radius of curvature is from about 0.5 mm to about 1.5 mm.

6. The stent-graft of any of claims 3 to 5 where the second radius of curvature is from about 4 mm to about 9 mm.

7. The stent-graft of claim 3 where a ratio of the first radius of curvature to the second radius of curvature is about 1:2.6 to about 1:18.

8. The stent-graft of any of claims 3 to 7 where at least one distal apex (804b) of the second stent ring (800b) comprises a third curved portion having a third radius of curvature, and at least one proximal apex (802b) of the second stent ring (800b) comprises a fourth curved portion having a fourth radius of curvature, where the fourth radius of curvature is greater than the third radius of curvature.

9. The stent graft of any preceding claim including at least one additional stent.

## Patentansprüche

1. Stent-Graft (800) für die Verwendung bei einem medizinischen Verfahren, wobei der Stent-Graft Folgendes umfasst:
einen Graft mit einem proximalen und einem distalen Ende (822, 824) und einem sich dazwischen erstreckenden Lumen (825),
einen ersten Stentring (800a), der eine Vielzahl von proximalen Scheiteln (802a), eine Vielzahl von distalen Scheiteln (804a) und eine Vielzahl von allgemein geraden Abschnitten umfasst, die zwischen der Vielzahl von proximalen und distalen Scheiteln angeordnet sind, wobei die Reihe von distalen Scheiteln (804a) des ersten Stentrings jeweils distal zum proximalen Ende (822) des Grafts angeordnet sind und wobei die Reihe von proximalen Scheiteln (802a) des ersten Stentrings jeweils proximal jenseits des proximalen Endes (822) des Grafts angeordnet sind, und
einen zweiten Stentring (800b), der eine Vielzahl von proximalen Scheiteln (802b), eine Vielzahl von distalen Scheiteln (804b) und eine Vielzahl von allgemein geraden Abschnitten umfasst, die zwischen der Vielzahl von proximalen und distalen Scheiteln angeordnet sind, wobei die Reihe von distalen Scheiteln (804b) des zweiten Stentrings jeweils distal zum proximalen Ende (822) des Grafts angeordnet sind und wobei die Reihe von proximalen Scheiteln (802b) des zweiten Stentrings jeweils proximal jenseits des proximalen Endes (822) des Grafts angeordnet sind,
wobei der erste (800a) und der zweite (800b) Stentring phasenverschoben sind,
wobei der erste Stentring (800a) ein erstes einheitliches Segment umfasst und der zweite Stentring (800b) ein zweites einheitliches Segment umfasst, wobei das erste einheitliche Segment Abschnitte umfasst, die sowohl intern als auch extern zu dem zweiten einheitlichen Segment angeordnet sind, und wobei sich der erste und der zweite Stentring gegenseitig überlappen, und
wobei der erste (800a) und der zweite (800b) Stentring, die sich überlappen, nur am proximalen Ende des Grafts angeordnet sind.

2. Stent-Graft nach Anspruch 1, wobei der erste Stentring (800a) und der zweite Stentring (800b) identische Geometrien umfassen.

3. Stent-Graft nach einem der vorhergehenden Ansprüche, wobei mindestens ein distaler Scheitel (804a) des ersten Stentrings (800a) einen ersten gekrümmten Abschnitt mit einem ersten Krümmungsradius umfasst und mindestens ein proximaler Scheitel (802a) des ersten Stentrings (800a) einen zweiten gekrümmten Abschnitt mit einem zweiten Krümmungsradius umfasst, wobei der zweite Krümmungsradius größer als der erste Krümmungsradius ist.

4. Stent-Graft nach Anspruch 3, wobei der zweite Krümmungsradius mindestens doppelt so groß wie der erste Krümmungsradius ist.

5. Stent-Graft nach einem der Ansprüche 3 und 4, wobei der erste Krümmungsradius zwischen ungefähr 0,5 mm und ungefähr 1,5 mm beträgt.

6. Stent-Graft Stent-Graft nach einem der Ansprüche 3 bis 5, wobei der zweite Krümmungsradius zwischen ungefähr 4 mm und ungefähr 9 mm beträgt.

7. Stent-Graft nach Anspruch 3, wobei ein Verhältnis des ersten Krümmungsradius zum zweiten Krümmungsradius ungefähr 1:2,6 bis ungefähr 1:18 beträgt.

8. Stent-Graft nach einem der Ansprüche 3 bis 7, wobei mindestens ein distaler Scheitel (804b) des zweiten Stentrings (800b) einen dritten gekrümmten Abschnitt mit einem dritten Krümmungsradius umfasst und mindestens ein proximaler Scheitel (802b) des zweiten Stentrings (800b) einen vierten gekrümmten Abschnitt mit einem vierten Krümmungsradius umfasst, wobei der vierte Krümmungsradius größer als der dritte Krümmungsradius ist.

9. Stent-Graft nach einem der vorhergehenden Ansprüche, einschließlich mindestens eines zusätzlichen Stents.

## Revendications

1. Stent couvert (800) destiné à être utilisé dans le cadre d'une procédure médicale, le stent couvert comprenant :
un implant comportant des extrémités proximale et distale (822, 824) et une lumière (825) s'étendant entre celles-ci ;
un premier anneau de stent (800a) comprenant une pluralité de saillies proximales (802a), une pluralité de saillies distales (804a), et une pluralité de parties globalement droites disposées entre la pluralité de saillies proximales et distales, les saillies de la série de saillies distales (804a) du premier anneau de stent étant chacune disposées en position distale vis-à-vis de l'extrémité proximale (822) de l'implant, et les saillies de la série de saillies proximales (802a) du premier anneau de stent étant chacune disposées en position proximale vis-à-vis de l'extrémité proximale (822) de l'implant ; et
un deuxième anneau de stent (800b) comprenant une pluralité de saillies proximales (802b), une pluralité de saillies distales (804b), et une pluralité de parties globalement droites disposées entre la pluralité de saillies proximales et distales, les saillies de la série de saillies distales (804b) du deuxième anneau de stent étant chacune disposées en position distale vis-à-vis de l'extrémité proximale (822) de l'implant, et les saillies de la série de saillies proximales (802b) du deuxième anneau de stent étant chacune disposées en position proximale au-delà de l'extrémité proximale (822) de l'implant ;
dans lequel le premier (800a) et le deuxième (800b) anneau de stent sont décalés ;
le premier anneau de stent (800a) comprenant un premier segment uniforme, et le deuxième anneau de stent (800b) comprenant un deuxième segment uniforme, le premier segment uniforme comprenant des parties disposées à la fois à l'intérieur et à l'extérieur du deuxième segment uniforme et les premier et deuxième anneaux de stent se chevauchant l'un l'autre ; et
dans lequel les premier (800a) et deuxième (800b) anneaux de stent en chevauchement sont situés uniquement au niveau de l'extrémité proximale de l'implant.

2. Stent couvert selon la revendication 1, dans lequel le premier anneau de stent (800a) et le deuxième anneau de stent (800b) comprennent des géométries identiques.

3. Stent couvert selon l'une quelconque des revendications précédentes, dans lequel au moins une saillie distale (804a) du premier anneau de stent (800a) comprend une première partie courbée présentant un premier rayon de courbure, et au moins une saillie proximale (802a) du premier anneau de stent (800a) comprend une deuxième partie courbée présentant un deuxième rayon de courbure, le deuxième rayon de courbure étant supérieur au premier rayon de courbure.

4. Stent couvert selon la revendication 3, dans lequel le deuxième rayon de courbure est égal au moins au double du premier rayon de courbure.

5. Stent couvert selon l'une quelconque des revendications 3 et 4, dans lequel le premier rayon de courbure est d'environ 0,5 mm à environ 1,5 mm.

6. Stent couvert selon l'une quelconque des revendications 3 à 5, dans lequel le deuxième rayon de courbure est d'environ 4 mm à environ 9 mm.

7. Stent couvert selon la revendication 3, dans lequel un rapport du premier rayon de courbure au deuxième rayon de courbure est d'environ 1:2,6 à environ 1:18.

8. Stent couvert selon l'une quelconque des revendications 3 à 7, dans lequel au moins une saillie distale (804b) du deuxième anneau de stent (800b) comprend une troisième partie courbée présentant un troisième rayon de courbure, et au moins une saillie proximale (802b) du deuxième anneau de stent (800b) comprend une quatrième partie courbée présentant un quatrième rayon de courbure, le quatrième rayon de courbure étant supérieur au troisième rayon de courbure.

9. Stent couvert selon l'une quelconque des revendications précédentes, comprenant au moins un stent supplémentaire.
